# EUROPEAN PATENT APPLICATION

(11) **EP 0 714 640 A1**
(43) Date of publication of application: **05.06.1996**
(21) Application number: 95308483.7
(22) Date of filing: 27.11.1995
(51) Int. Cl.: A61F 2/06

(54) **System and method for delivering multiple stents**

(30) Priority: 28.11.1994 US 345488
(71) Applicant: ADVANCED CARDIOVASCULAR SYSTEMS, INC., Santa Clara California 95052 (US)
(72) Inventor: Stack, Richard S., Chapel Hill, North Carolina 27514 (US); Williams, Michael S., Chapel Hill, North Carolina 27516 (US)
(74) Representative: Mayes, Stuart David

(57) **Abstract**

Stent segments can be aligned, and delivered spaced apart or overlapped within a body lumen. In one embodiment, an elongated catheter (12) is provided with a plurality of expandable balloon members (16), each of which is adapted to receive an expandable stent segment for delivery and release in a body lumen. In another embodiment, each balloon member is selectively expandable. In another embodiment, an inflatable slotted dilatation catheter balloon is provided that maintains the stent segments placed thereon aligned and spaced apart as desired during expansion of the balloon. Stent segments are provided having a plurality of flow passages therethrough to allow for side branch vessel perfusion, cell growth, flexibility, oxygenation and nutrient exchange to the blood vessel wall, and to present a reduced surface area in order to reduce thrombogenesis.

## Description

### BACKGROUND OF THE INVENTION

### FIELD OF THE INVENTION

This invention relates generally to the treatment of cardiovascular diseases such as coronary artery stenosis, restenosis, acute thrombosis, intimal hyperplasia and sub-acute thrombosis. More particularly, the invention concerns delivery and release of multiple stents.

### DESCRIPTION OF RELATED ART

Coronary angioplasty is a medical procedure which is useful for treatment of atherosclerotic vascular disease. In a typical percutaneous transluminal coronary angioplasty (PTCA) procedure, a guiding catheter is introduced into the cardiovascular system through a brachial or femoral artery, and is advanced toward the area of the vasculature to be treated. A dilatation catheter having an inflatable angioplasty balloon can be introduced through the guiding catheter by sliding the dilatation catheter over a guide wire disposed in a lumen of the dilatation catheter. The guide wire is advanced through the guiding catheter out of the distal end of the guiding catheter, and is maneuvered into position in the vasculature of the patient up to and beyond the lesion to be treated. The dilatation catheter then is advanced over the guide wire until the angioplasty balloon is located within and across the lesion. The balloon then can be inflated with inflation fluid to a pre-determined size to dilate the portion of the vasculature being treated. The balloon then is deflated, and the dilatation catheter removed to allow blood flow to resume.

By way of example, further details of the angioplasty procedure and the devices used in such a procedures can be found in U.S. Patent No. 4,323,071; U.S. Patent No. 4,439,185; U.S. Patent No. 4,516,972; U.S. Patent No. 4,538,622; U.S. Patent No. 4,554,929; U.S. Patent No. 4,616,652; U.S. Patent No. 4,638,805; and U.S. Patent No. 4,748,982.

Restenosis at or near the site of the original lesion or stenosis in the blood vessel is a common problem that can occur after angioplasty, and can require a second angioplasty procedure or bypass surgery. Stents offer a mechanical approach to treating either an original stenosis or restenosis and the associated problems of intimal hyperplasia and mechanical recoil of the treated vessel.

Single-unit locking stents are known that can be delivered with balloon catheters, by positioning the stents over the balloon portion of the catheter, and by expanding the stents with the balloon from a reduced diameter to an enlarged diameter that is greater than or equal to the diameter of the blood vessel wall to be treated. Examples of expandable catheters and stents are disclosed in U.S. Patent No. 5,102,417; U.S. Patent No. 5,123,917; and U.S. Patent No. 5,133,732.

It sometimes is desirable to implant more than one stent at an area of lesion in a blood vessel, particularly where the lesion or lesions extend over an area encompassing one or more side branches from the blood vessel. Because implantation of a stent in a vessel at a location where a side branch vessel exists can block or otherwise interfere with blood flow through the side branch, it would be desirable to provide a system for delivering multiple, independent stent segments that can be Placed on either side of a side branch vessel, or merely for placing multiple stent segments within an extended and specifically selected area of lesion in a vessel.

Dilatation catheter balloons can inflate unevenly, particularly in the vasculature at an area of lesion or stenosis, such that multiple stent segments placed on a dilatation catheter balloon can become misaligned or improperly spaced. While uneven expansion of a balloon with a single stent can alter the expansion and performance of the stent as a whole, placement of the stent typically can be adjusted. Misalignment or slippage of multiple stents, particularly at a location of a side branch can present a problem of blockage or interference with blood flow at the side branch.

It therefore would be desirable to provide a system for delivering multiple stent segments to a location in the vasculature of a patient to be treated that would maintain the alignment and the spacing of the multiple stent segments for proper placement of the stent segments. It also would be desirable to provide a system and method for placing multiple stents either spaced apart or overlapping one another. It also would be desirable to provide stent segments that would allow for side branch flow, cell growth, flexibility, and oxygenation and nutrient exchange to the vessel wall, and which have a decreased surface area in order to be less thrombogenic.

### SUMMARY OF THE INVENTION

Briefly, certain embodiments of the present invention provide for systems and methods for delivering multiple stent segments in a body lumen that permit the stent segments to be aligned so as to be spaced apart or to be overlapping, as desired. In one currently preferred embodiment, an elongated catheter is provided with a plurality of expandable balloon members, each of which is adapted to receive an expandable stent segment for delivery and release in a body lumen. In another currently preferred embodiment, each balloon is selectively expandable. In another currently preferred embodiment, an inflatable slotted dilatation catheter balloon is provided that maintains the stent segments placed thereon aligned and spaced apart as desired during expansion of the balloon. In one currently preferred embodiment, the stent segments have a plurality of flow passages therethrough to allow for side branch vessel perfusion, cell growth, flexibility, oxygenation and nutrient exchange to the blood vessel wall, and to present a reduced surface area in order to reduce thrombogenesis.

Embodiments of the invention accordingly provide for a system for delivery of a plurality of expandable stents, comprising an elongated catheter having a plurality of expandable balloon members, each of which adapted to receive an expandable stent. In one currently preferred embodiment, each balloon member preferably is connected in fluid communication with a separate inflation lumen for receiving inflation fluid for expanding the balloon members, so that the balloon members can be expanded sequentially or in unison as desired, to allow delivery of the expandable stents either spaced apart or overlapping. An inner lumen also preferably is provided in the elongated catheter which is adapted to receive a guide wire that extends the entire length of the inner lumen.

In another currently preferred embodiment, the invention provides for a system for delivering a plurality of expandable stents, comprising an elongated catheter having an inflatable slotted dilatation catheter balloon for delivery of at least one cylindrical stent segment in a body lumen. The balloon preferably includes an inner lumen for receiving inflation fluid, and a balloon outer wall defining at least one exterior cylindrically-slotted portion for receiving a cylindrically-expandable stent segment. Each cylindrically-slotted portion preferably extends around the circumference of the balloon and is defined on either side of the radially cylindrical slot by a raised portion of the exterior wall of the balloon. In a currently preferred embodiment, the slotted dilatation catheter balloon includes a plurality of cylindrically-slotted portions extending around the circumference of the balloon.

Although various types of stent segments can be used with the stent delivery systems of the invention, in one currently preferred embodiment, the cylindrical stent segments can be of a locking design, including a head end portion, an elongated main body portion connected to the head end portion, and a tail end portion connected to the main body portion and opposing the head end portion. An elongated slot is disposed transversely in the head end portion' so that the tail end portion can be placed therethrough to interlock with the head end portion. Each cylindrical stent segment preferably has a surface defining a plurality of apertures therethrough to allow side branch blood perfusion, cell growth, flexibility, oxygenation and nutrient exchange. The cylindrical stent currently preferably comprises a metal structural member, and can be coated with one or more layers of polymeric material.

These and other aspects and advantages of the invention will become apparent from the following detailed description and the accompanying drawings, which illustrate by way of example the features of the invention.

### BRIEF DESCRIPTION OF THE DRAWINGS

FIGURE 1 is a partial longitudinal cross-sectional view of a first embodiment of a stent delivery system according to the invention;
FIG. 2 is a top view of a portion of the stent delivery system taken along line 2-2 of FIG. 1;
FIG. 3 is a cross-sectional view of the stent delivery system taken along line 3-3 of FIG. 1;
FIG. 4 is a cross-sectional view of the stent delivery system taken along line 4-4 of FIG. 1;
FIG. 5 is a cross-sectional view of the stent delivery system taken along line 5-5 of FIG. 1;
FIG. 6 is a partial cross-sectional view of the distal portion of an elongated catheter of a second embodiment of a stent delivery system similar to that of FIG. 1, but having individual inflation lumens for each of the plurality of balloon members;
FIG. 7 is a cross-sectional view through a balloon member and the elongated catheter of the stent delivery system of FIG. 6 taken along line 7-7 of FIG. 6;
FIG. 8 is an elevational view of the distal portion of the elongated catheter of FIG. 6 having a plurality of stent segments mounted on the balloon members of the catheter;
FIG. 9 is a cross-sectional view through a stent segment, balloon member and the elongated catheter taken along line 9-9 of FIG. 8;
FIG. 10 illustrates advancement of the stent delivery system of FIG. 6 with stent segments mounted as in FIG. 8, into an artery which has been damaged;
FIG. 11 illustrates inflation of the balloon members of the elongated intravascular catheter of FIG. 1 to expand the stent segments mounted on the balloon members;
FIG. 12 illustrates a plurality of expanded stent segments disposed in a damaged section of an artery maintaining the patency of the artery;
FIG. 13 is a top plan view of a first embodiment of a cylindrical locking stent segment for use with a stent delivery system embodying the invention;
FIG. 14 is a top plan view of another embodiment of the cylindrical locking stent segment for use a the stent delivery system embodying the invention;
FIG. 15 is an elevational view of an inflatable slotted dilatation catheter balloon for delivering cylindrical stent segments in a body lumen;
FIG. 16 is an elevational view of the inflatable slotted dilatation catheter balloon of FIG. 15 carrying multiple locking stent segments;
FIG. 17 is a partial longitudinal cross-sectional view of an inflatable slotted dilatation catheter balloon carrying multiple stents segments as in FIG. 16, connected to an elongated dilatation catheter, for delivery and release of the stent segments.

### DETAILED DESCRIPTION OF THE PREFERRED EMBODIMENTS

Placement of a stent at the location of a side branch blood vessel can block or interfere with blood flow through the side branch blood vessel. While placement of multiple stents on either side of the side branch blood vessel can avoid blood flow blockage through the side blood vessel, delivery of multiple stents on a balloon can be problematic due to uneven balloon expansion or misalignment or changes in the spacing of the stents on the balloon; and placement of a stent at the location of the side branch blood vessel still may be necessary.

As is shown in the drawings for purposes of illustration, in a first currently preferred embodiment an intravascular catheter 12 has an elongated catheter body 14 and a plurality of balloons 16a,b,c,d on the distal portion of the catheter body. The edges 15 of the balloons preferably can be provided with a relatively steep angle of taper, typically between 60° and 80°, to allow for a minimum spacing between balloons, such as about 0.5 millimeter, for example. Advancement markers also can be provided on the catheter 12, such as in increments of about 0.5 millimeter or greater, for example, to indicate forward tracking distance of each stent segment being deployed. A manipulating device 18 can be provided on the proximal end of the delivery system to effect relative axial or longitudinal movement of the intravascular catheter 12. A plurality of expandable stent segments to be delivered within a body lumen of a patient can be mounted on the exteriors of each balloons. The expandable stent segments preferably include a metal radiopaque structure, such as at the edges of the stent segments, to serve as one or more radiopaque markers will aid in alignment and positioning of the stent segments.

The intravascular catheter 12 has a distal port 20 and a proximal port 22 which are in fluid communication with a first inner lumen 24 extending within the distal portion of the catheter 12 and adapted to slidably receive a guidewire therein. The proximal end of the first inner lumen 24 is provided with a ramp 26 to guide the proximal end of a guidewire 28 out of the proximal port 22 of intravascular catheter 12 when the catheter is mounted onto the guidewire. A second, much longer inner lumen 30 is provided within the catheter body 14 to direct inflation fluid from the proximal end of the catheter body to the interiors 29 a,b,c,d of the balloons. The inflation lumen 30 communicates with the interiors of the balloons through the inflation ports 31 a,b,c,d.

Proximal to the proximal port 22 in the catheter body 14 is a stiffening member 32 which is disposed in an inner lumen 34 provided within the catheter body 14. As shown in the drawings, the inner lumen 34 and the first inner lumen 24 may be the same lumen.

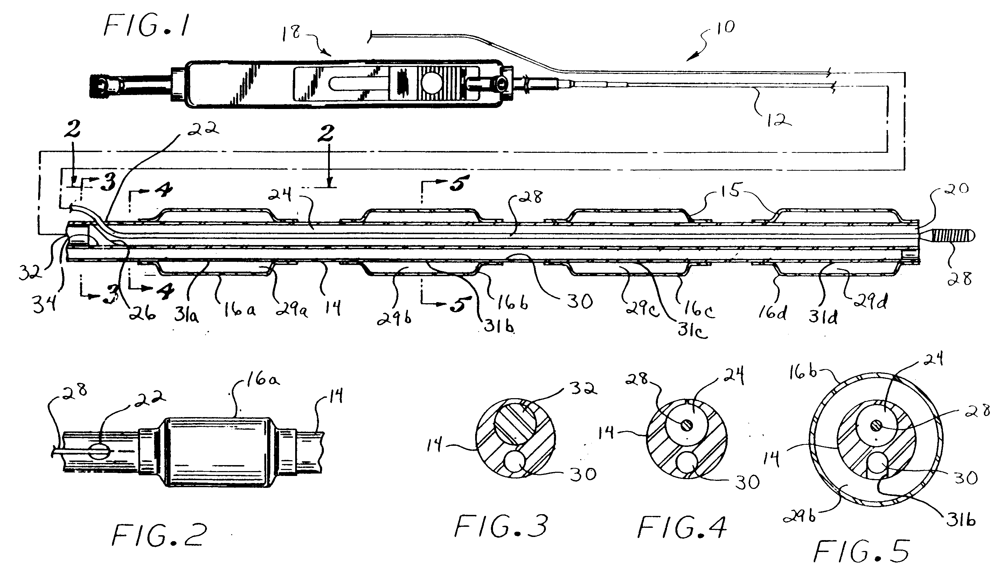

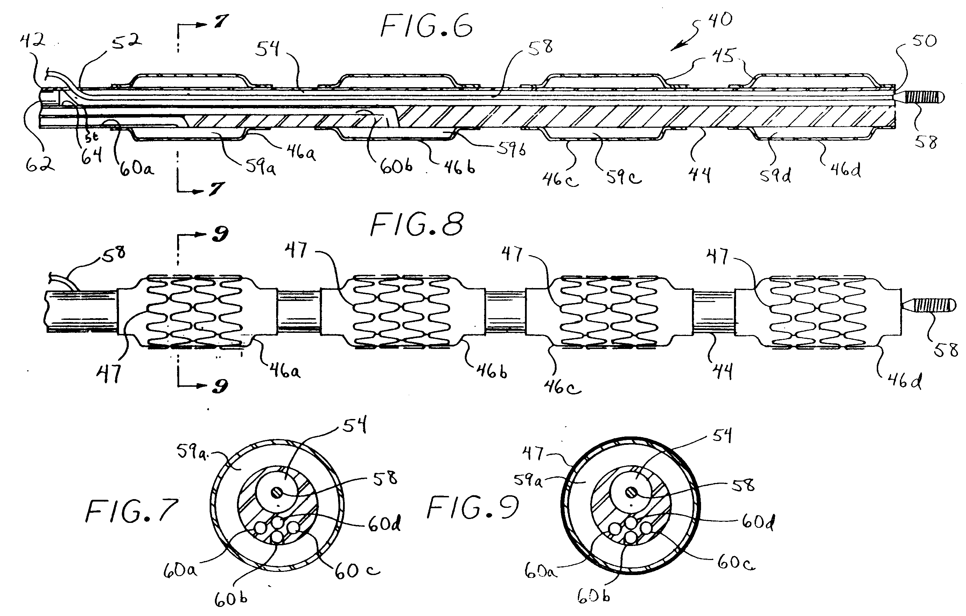

With reference to FIGS. 6-9, in a second currently preferred embodiment of a multiple stent delivery system, an intravascular catheter 42 has an elongated catheter body 44 and a plurality of balloons 46a,b,c,d on the distal portion of the catheter body. The edges 45 of the balloons preferably can have a relatively steep taper, such as between 60° and 80°, for example. The intravascular catheter can be connected to a manipulating device such as is illustrated in FIG. 1. As is shown in FIG. 6, an expandable stent 47 that is to be delivered within a body lumen of a patient can be mounted over the exterior of the balloons.

The intravascular catheter 42 has a distal port 50 and a proximal port 52 which are in fluid communication with a first inner lumen 54 extending within the distal portion of the catheter, and that is adapted to slidably receive a guidewire. The proximal end of the first inner lumen 54 is provided with a ramp 56 to guide the proximal end of the guidewire 58 out of the proximal port of the intravascular catheter when the catheter is mounted onto the guidewire. A plurality of inflation lumens, only two of which, lumens 60a and 60b, are shown in FIG. 6, are provided within the catheter body. Each lumen receives and directs inflation fluid from the proximal end of the catheter body to the interior, 59a,b,c,d of balloons 46a,b,c,d, so that the balloons can be selectively and independently inflated and deflated, to allow expandable stent segments 47 mounted thereon to be selectively and independently delivered and released within a body lumen, either spaced apart or overlapping. Inflation lumens for the most distal two balloons are not shown in FIG. 6 for convenience of illustration. Where the stent segments are to be overlapped, the stent segments also can have tapered edges to reduce buildup of the profile of the stent segments.

Proximal to the port 52 in the catheter body is a stiffening member 62 that is disposed in the inner lumen 64 within the catheter body. As is illustrated, the inner lumen 64 and inner lumen 54 may be the same lumen.

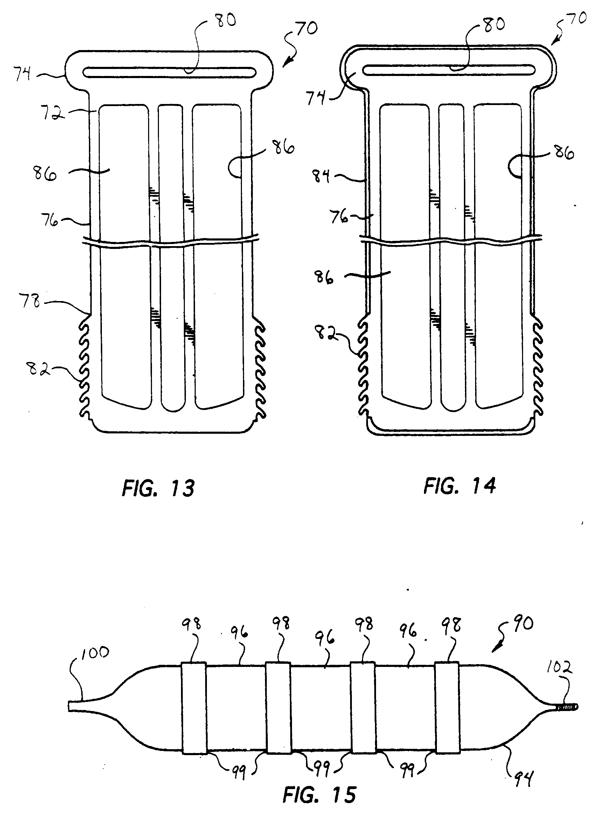

In a third currently preferred embodiment, the invention provides for an inflatable slotted dilatation catheter balloon for delivery of one or more cylindrical stent segments in a body lumen. In the third embodiment of a system for delivering the cylindrical stent segments, the stent segments preferably are individual locking cylindrical stent segments adapted to be carried in slots of the slotted dilatation catheter balloon, although other types of expandable stent segments also can be used. With reference to FIGS. 13 and 14, the cylindrical, locking stent segments 70 typically comprise a main structural member 72 that can be made, for example, of a metal or a polymer. The main structural member can be made from metals such as stainless steel, tantalum, nickel titanium alloy (or "nitinol"), platinum iridium alloy, molybdenum-rhenium alloy, gold, magnesium, combinations thereof and other similar metals. A sheet of the thin metal can be cut in the desired shape to form the metal structural member with a laser, such as a continuous CO₂ laser, a pulsed YAG (yttrium aluminum garnet) laser, an excimer laser, for example, or alternatively, by chemical etching or stamping.

The main structural member of the stent currently preferably is formed to include a proximal or head end 74 and a main body portion 76 connected to the head end and extending distally from the head end. The main body portion includes a distal or tail end portion 78 opposing the head end. The head end of the stent segments 70 preferably includes an elongated aperture or slot 80 corresponding to the lengthwise dimension of the main body portion. The main structural member 72 preferably is formed so as to be capable in being rolled up into a cylindrical configuration whereby the tail end portion of the locking stent segment can be inserted through the corresponding slot in the head end. In a currently preferred embodiment, at each side of the tail end portion, the periphery of the tail end portion of the main structural member includes a plurality of pronged teeth 82 generally projecting outward and distally from the head end. As is shown in FIG. 14, the main structural member of the locking stent segment can also be laminated on one or both sides with a layer of a polymer 84, having a polymeric matrix that can be loaded with and can release therapeutic drugs.

The polymeric material with which the main structural member of the cylindrical locking stent can be laminated preferably comprises a biodegradable, bioabsorbable polymeric film that is capable of being loaded with therapeutic drugs and then releasing the same. The polymeric materials preferably include, but are not limited to, polycaprolactone, poly-DL-lactic acid (DL-PLA) and poly-L-lactic acid (LPLA) or lactate. Other biodegradable, bioabsorbable polymers, such as polyorthoesters, polyiminocarbonates, polydioxanone, poly (ethylene oxide), polyvinylpyrrolidone, hydroxyethyl cellulose, polyvinyl alcohol, polyamides, polyhydroxybutyric acid, collagen, and linear aliphatic polyesters also may be suitable, and other non-degradable polymers such as polyethylene terephthalate, and polycarbonates, for example, that are capable of carrying and delivering therapeutic drugs also may be suitable.

The main body portion 76 of the main structural member 72 also preferably includes a plurality of apertures 86 extending entirely through the stent segment, to allow blood flow through the stent to side branch vessels and to allow blood flow to the vessel wall, such as for oxygenation of and nutrient exchange with the vessel wall, and in order to present a decreased surface area for purposes of reducing thrombogenicity of the stent segment 70. The apertures also improve the flexibility of the stent segment, allowing the stent segment to be rolled, and also facilitate the process of cell growth over the surface of the stent segment.

With reference to FIGS. 15, 16 and 17, the system also provides for a slotted dilatation catheter balloon 90, having an interior inflation lumen 92 for receiving inflation fluid. The balloon can be either a single-lumen or a dual-lumen balloon, but is shown here as a currently preferred, typical dual-lumen balloon. The balloon includes an exterior balloon wall 94 formed to define cylindrical, circumferential exterior slots or depressed portions 96 for receiving the aforementioned cylindrical locking stent segments. The balloon provides at least one slot for receiving a cylindrical locking stent segment, but preferably includes a plurality of slots, for delivering and releasing a plurality of cylindrical locking stent segments. The exterior balloon wall preferably is shaped to define raised portions 98 on either side of each slot, to provide raised shoulder areas 99 for retaining the alignment and spacing of the cylindrical locking stent segments on the balloon until the stent segments are expanded, delivered and released. Other types of cylindrical locking stent segments also may be suitable for delivery and release by the slotted dilatation catheter balloon in this manner.

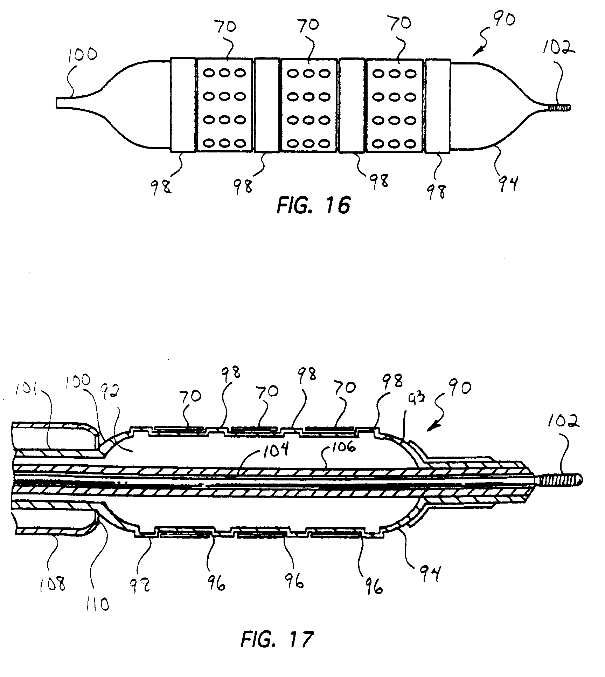

As is illustrated in FIG. 17, the slotted dilatation catheter balloon is connected at the proximal end 100 of the balloon 93 to intravascular catheter 101, and is guided over a guidewire 102 extending through the balloon, and disposed in an inner guidewire lumen 104 of an inner guide wire wall member 106. The slotted dilatation catheter balloon can be protected in a sheath 108 until it is moved to the location of the blood vessel to be treated, at which time the balloon and the locking stent segments that are carried by the slotted balloon can be moved out of the sheath through a port 110 in the sheath.

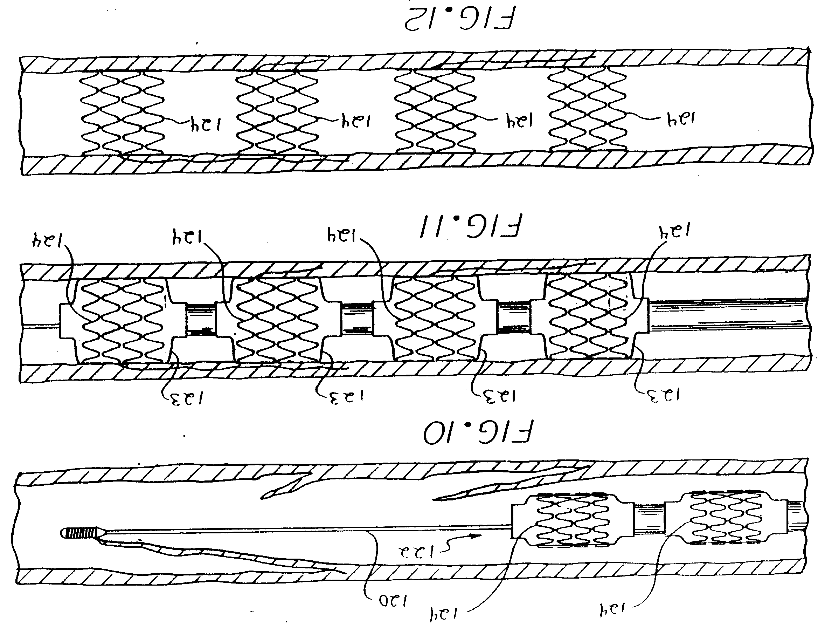

With reference to FIGS. 10-12, in a typical situation, the stent delivery system can be used when the arterial lining of a patient has been damaged to such an extent that the lining requires support to prevent it from collapsing into the arterial passageway and thereby obstructing sufficient blood flow through the blood vessel. When the damage occurs during an intravascular procedure, there typically will be a guidewire 120 (or other guiding member) in place extending across the damaged section of the artery such as is shown in FIG. 10. The proximal end of the guidewire extends out of the patient during the entire procedure and is inserted through the catheter 122.

The intravascular catheter 122 can be positioned within a delivery sheath 108 as illustrated in FIG. 17 for delivery to the location of the lesion to be treated. The proximal end of the guidewire 120 then manually may be held to maintain the position of the guidewire within the vasculature while the stent delivery system is advanced over the guidewire and into the patient. When the balloon 123 and the multiple stent segments 124 mounted thereon are properly placed within the damaged artery, inflation fluid is directed under substantial pressure through at least one inflation lumen in the catheter body to the interior of at least one balloon, expanding that balloon and simultaneously expanding at least one of the stents against the blood vessel wall, as is shown in FIG. 11. When selectively-expandable multiple balloons are used, the catheter can be advanced after delivery of a stent segment at one site of treatment, and then incrementally advanced using markers on the stent segments and incremental markers on the catheter shaft, to deliver additional stent segments either spaced apart or overlapping sequentially, as desired. The delivery system then may be removed from the patient along with the guidewire, leaving one or more of the expanded stents within the damaged arterial section as shown in FIG. 12 to maintain the patency of the arterial section.

It thus has been demonstrated that the system provides for an inflatable slotted dilatation catheter balloon for delivery of at least one cylindrical locking stent segment in a body lumen, allowing for proper alignment and spacing of such stent segments, and for a system for delivering at least one cylindrical locking stent segment in a body lumen, comprising in combination the inflatable slotted dilatation catheter balloon and at least one cylindrical locking stent segment that can allow for blood flow through side branch blood vessels, cell growth, and oxygenation and nutrient exchange to a blood vessel wall. The slotted dilatation catheter balloon and the system overcome the problem of uneven expansion of a stent delivery balloon that disrupts proper placement of stents in a body lumen. The same or different types of stent segments can be used with the systems and methods described for delivering multiple stents, and different stent segments can be loaded with different types of drugs to treat different areas of a lesion. Since multiple stent segments can be mounted for delivery, the stiffening effects of the stent segments on the catheter and the body lumen can be minimized.

The dimensions of the intravascular catheter generally will follow the dimensions of intravascular catheters used in angioplasty procedures in the same arterial location. Typically, the length of a catheter for use in the coronary arteries is about 150 centimeters, the outer diameter of the catheter shaft is about .89 millimeter (0.035 inch), the length of the balloon typically is about 2 centimeters, although the length can vary from about 1 millimeter to about 60 millimeters or more, and the inflated diameter about 1 to about 8 millimeters although an inflated diameter of about 2-4 millimeters is currently preferred.

The materials of construction may be selected from those used in conventional balloon angioplasty catheters, such as those described in the patients incorporated by reference. The delivery sheath generally will be slightly shorter than the intravascular catheter, e.g., by about the length of the manipulating device, but will be long enough to cover the balloon and the stent segments, with an inner diameter large enough to accommodate the intravascular catheter and to allow the catheter free longitudinal movement therein. The sheath and the catheter shaft can be made of conventional polyethylene tubing or another suitable material.

While the present invention has been described herein in terms of delivering an expandable stent to a desired location within a blood vessel of a patient, the systems and method of delivery can be employed to deliver stents to locations within other body lumens, such as the urethra or the Fallopian tubes so that the stents can be expanded to maintain the patency of these body lumens. Various changes and improvements also may be made to the invention without departing from the scope thereof.

## Claims

1. A system for the delivery of a plurality of expandable stent segments within a body lumen, comprising:
an elongated catheter having proximal and distal ends, said elongated catheter having at least one expandable balloon member adjacent to the distal end of the elongated catheter adapted to receive on the exterior thereof a plurality of expandable stent segments.

2. The stent delivery system of claim 1 wherein the elongated catheter further comprises a plurality of expandable balloon members, and a plurality of inflation lumens in fluid communication with said plurality of expandable balloon members, said inflation lumens being adapted to selectively communicate an inflation fluid with said expandable members to selectively expand said expandable balloon member.

3. The stent delivery system of claim 2 wherein the elongated catheter further comprises an inner lumen adapted to receive a guiding member therein and which extends the entire length of said inner lumen.

4. A system for the delivery of a plurality of expandable stent segments within a body lumen, comprising:
an elongated catheter having proximal and distal ends, said elongated catheter having a plurality of selectively expandable balloon members adjacent to the distal extremity of the elongated catheter, said expandable balloon members being adapted to receive on the exterior thereof an expandable stent segment, said elongated catheter having an inner lumen which is adapted to slidably receive a guiding member therein, said inner lumen extending between a first port in the distal extremity of said catheter and a second port proximal to said expandable balloon members.

5. The stent delivery system of claim 4 wherein the elongated catheter further comprises:
a plurality of inflation lumens in fluid communication with said plurality of expandable balloon members, said inflation lumens being adapted to selectively communicate inflation fluid to said expandable balloon members to selectively expand said expandable members.

6. A system for delivering stent segments within a body lumen, comprising:
an inflatable slotted dilatation catheter balloon for delivery of at least one cylindrical stent segment in the body lumen;
an inner lumen provided in said balloon for receiving inflation fluid for inflation of the balloon; and
an interior balloon wall defining at least one exterior circumferential slotted portion for receiving and aligning at least one cylindrical expandable stent segment.

7. The system of claim 6, wherein each circumferential slotted portion has a raised portion on either side of the circumferential slotted portion.

8. The system of claim 6, wherein said balloon wall defines a plurality of circumferential slotted portions.

9. A system for delivering at least one cylindrical stent segment in a body lumen, comprising in combination:
an expandable slotted balloon having an inner lumen for receiving inflation fluid for inflation of the balloon;
an exterior balloon wall defining at least one exterior circumferential slotted portion for receiving and aligning at least one cylindrical expandable stent segment; and
at least one cylindrical expandable stent segment.

10. The system of claim 9, wherein said cylindrical stent segment comprises a cylindrical locking stent segment.

11. The system of claim 10, wherein said cylindrical locking stent segment comprises:
a proximal head end portion;
an elongated main body portion connected to said head end portion, said main body portion extending distally from said head end portion, said main body portion having a tail end portion opposing said head end portion; and
an elongated aperture disposed in said head end portion extending transversely to said elongated main body portion, said tail end portion being adapted to interlock with said head end portion through said elongated aperture in said head end portion.

12. The system of claim 10, wherein each said cylindrical locking stent segment has a surface defining a plurality of apertures in said main body portion allowing for blood flow therethrough.

13. The system of claim 10, wherein said cylindrical locking stent segment comprises a main metal structural member.

14. The system of claim 13, wherein said main metal structural member is coated with a layer of polymeric material capable of being loaded with and releasing therapeutic agents in a blood vessel.

15. A method for delivering a plurality of expandable stent segments within a body lumen, comprising the steps of:
advancing an elongated catheter having a plurality of selectively expandable balloon members with a plurality of expandable stent segments mounted thereon at a distal end thereof within a body lumen to position a first balloon member of the plurality of balloon members at an area within the body lumen to be treated,
selectively expanding the first expandable balloon member to deliver a first stent segment of said plurality of expandable stent segments within the body lumen;
advancing the elongated catheter within the body lumen to position a second balloon member of the plurality of balloon members at an area within the body lumen to be treated; and
selectively expanding the second balloon member to deliver a second stent segment of said plurality of expandable stent segments within the body lumen.

16. The method of claim 15, wherein the first and the second expandable stent segments are delivered in spaced apart relation to one another.

17. The method of claim 15, wherein the first and the second expandable stent segments are delivered in overlapping relation to one another.
